# EUROPEAN PATENT APPLICATION

(11) **EP 0 640 576 A1**
(43) Date of publication of application: **01.03.1995**
(21) Application number: 94202420.9
(22) Date of filing: 24.08.1994
(51) Int. Cl.: C07C 5/27

(54) **Process for the isomerisation of a hydrocarbonaceous feedstock**

(30) Priority: 25.08.1993 EP 93202499
(71) Applicant: SHELL INTERNATIONALE RESEARCH MAATSCHAPPIJ B.V., NL-2596 HR Den Haag (NL)
(72) Inventor: Dogterom, Ronald Jan, NL-1031 CM Amsterdam (NL); Jongkind, Hermanus, NL-1031 CM Amsterdam (NL); Kraushaar-Czarnetzki, Bettina, NL-1031 CM Amsterdam (NL)

(57) **Abstract**

Process for the isomerisation of a hydrocarbonaceous feedstock substantially boiling in the gasoline range which feedstock comprises linear paraffins having at least five carbon atoms by contacting the feedstock in the presence of hydrogen at elevated temperature and pressure with a catalyst comprising a MeAPO and/or MeAPSO medium-pore molecular sieve and at least one Group VIII metal component, wherein Me is at least Mg, Mn, Co or Zn.

## Description

The present invention relates to a process for the isomerisation of a hydrocarbonaceous feedstock substantially boiling in the gasoline range which feedstock comprises linear paraffins having at least five carbon atoms.

One of the main objects in nowaday's oil refining is to produce gasolines fulfilling the increasing environmental demands on product quality and having a high octane number.

This means for gasoline that the octane specification has now to be established without lead-containing additives, less aromatics, in particular benzene, less olefins and lower gasoline vapour pressure.

Components which improve the octane quality of gasoline include branched paraffins. Object of the present invention is to provide branched paraffins.

Therefore, the present invention relates to a process for the isomerisation of a hydrocarbonaceous feedstock substantially boiling in the gasoline range which feedstock comprises linear paraffins having at least five carbon atoms by contacting the feedstock in the presence of hydrogen at elevated temperature and pressure with a catalyst comprising a MeAPO and/or MeAPSO medium-pore molecular sieve and at least one Group VIII metal component, wherein Me is at least one of Mg, Mn, Co or Zn.

In this way a very attractive high yield of branched paraffins, predominantly existing of mono-branched paraffins, can be obtained. The mono-branched paraffins obtained in accordance with the present process can as such suitably be applied for the production of multibranched paraffins, which constitute even more attractive high octane gasoline components.

In the context of the present invention a MeAPO medium-pore molecular sieve is defined as a crystalline microporous metal aluminophosphate composition having a three-dimensional framework structure of interconnected MeO₂, AlO₂ and PO₂ tetrahedral units, which framework structure has a medium-pore size. This type of molecular sieve and its preparation have for instance been described in US 4,567,029 which is hereby incorporated by reference.

Moreover, in the context of the present invention a MeAPSO molecular sieve is defined as a crystalline microporous metal silicoaluminophosphate composition having a three-dimensional framework structure of interconnected MeO₂, AlO₂, SiO₂ and PO₂ tetrahedral units, which framework structure has a medium-pore size. MeAPSO molecular sieves and their preparation have for example been disclosed in EP 158348, EP 158975, EP 161489 and EP 161490 which are hereby incorporated by reference.

An important advantage of the catalysts to be used in the present process is that they display a high selectivity towards isomerisation and at the same time a low selectivity towards cracking.

Preferably, the hydrocarbonaceous feedstock is contacted with a catalyst comprising a MeAPO and/or MeAPSO medium-pore molecular sieve and at least one Group VIII metal component, wherein Me is at least Co.

Suitable CoAPO and CoAPSO catalysts include CoAPO-11 and CoAPSO-11 catalysts.

In the context of the present invention a medium-pore size is defined as an average pore diameter in the range of 3.8 to 7 Å, preferably in the range of 4 to 6.5 Å.

Suitably, the medium-pore molecular sieve(s) has (have) an anhydrous composition which can be expressed in terms of molar oxide ratios as follows: (MeO)ₐ(Al₂O₃)_{b}(P₂O₅)_{c}(SiO₂)_{d}, wherein a ranges from 0.003 to 0.2, preferably from 0.005 to 0.15, b ranges from 0.05 to 0.3, preferably from 0.05 to 0.27, c ranges from 0.05 to 0.3, preferably from 0.05 to 0.27, and d is at most 0.4, preferably at most 0.3.

The hydrocarbonaceous feedstock substantially boiling in the gasoline range can suitably be obtained by fractionating any paraffinic gasoline-containing hydrocarbonaceous feedstock. Preferably the feedstock substantially boiling in the gasoline range is obtained by subjecting a straight run hydrocarbon oil to a fractionation, preferably atmospheric distillation. Other suitable hydrocarbonaceous feedstocks substantially boiling in the gasoline range include product fractions obtained from cracking processes such as catalytic cracking, thermal cracking, delayed coking, visbreaking and flexicoking. Hydrocarbonaceous feedstocks containing unacceptable levels of sulphur and nitrogen may be subjected to a hydrotreatment before they are subjected to the process according to the present invention, whereby particularly advantageous results may be obtained. Suitably the hydrocarbonaceous feedstock boiling in the gasoline range has a low sulphur content, preferably less than 10 ppmw on feed. The hydrocarbonaceous feedstock may consist entirely of a fraction boiling in the gasoline range, i.e. in the range of C₄ - 220 °C. While the full gasoline boiling range fraction may be included in the hydrocarbonaceous feedstock, it may be preferred to employ as hydrocarbonaceous feedstock a cut thereof having a boiling point up to 180 °C.

Suitably, the hydrocarbonaceous feedstock substantially boiling in the gasoline range may substantially comprise linear paraffins having at least five carbon atoms, i.e. the feedstock may substantially consist of one or more different types of linear paraffins having at least five carbon atoms.

Preferably, the hydrocarbonaceous feedstock boiling in the gasoline range substantially comprises linear paraffins having five to ten carbon atoms. More preferably, the hydrocarbonaceous feedstock substantially comprises linear paraffins having six to eight carbon atoms. Suitably, the hydrocarbonaceous feedstock substantially comprises n-heptane. Apart from n-heptane the feedstock may contain small amounts of C₇ naphthenes and mono-branched C₇ paraffins.

Process conditions which can be suitably applied, comprise a temperature of 200 to 450 °C, a total pressure of up to 100 bar, a space velocity of 0.5 to 10 kg/kg.h and presence of hydrogen at a H₂/feedstock weight ratio of less than 5. Preferred conditions comprise a temperature of 250 to 400 °C, a pressure of 2 to 50 bar, a space velocity of 1 to 10 kg/kg.h and presence of hydrogen at a H₂/feedstock weight ratio of greater than zero and less than 2.

Suitably, unconverted linear paraffins are separated from branched paraffins downstream the reaction zone wherein the isomerisation process is carried out, and recycled to the reaction zone. Such a separation can suitably be carried out by means of a molecular sieve or by means of distillation.

The catalyst to be used in accordance with the present invention comprises in addition to the MeAPO and/or MeAPSO medium-pore molecular sieve at least one Group VIII metal component. The catalyst may further comprise a binder material comprising one or more refractory oxides. The Group VIII metal-component may be present on the medium-pore molecular sieve structure or may be present on a separate carrier (e.g. the binder) comprising, for instance, one or more refractory oxides, e.g. alumina. Preferably, the Group VIII metal component is present on the molecular sieve structure. Suitably, the Group VIII metal component is loaded on the molecular sieve structure by means of ion-exchange or impregnation, although also other methods known in the art may be applied. Suitably, the Group VIII metal component is distributed in the molecular sieve structure. Preferably, a substantial amount of the Group VIII metal component is in metallic form which can be achieved by reduction procedures known in the art. Most preferably, the entire amount of the Group VIII metal component is in metallic form. Preferably, the Group VIII metal component comprises a noble-metal component, more preferably a platinum and/or palladium component. Suitably, the catalyst comprises 0.01 to 5% by weight of Group VIII metal based on total catalyst. More preferably, the catalyst comprises 0.1 to 3% by weight of Group VIII metal based on total catalyst.

The invention will now be illustrated by means of the following Examples.

### Example 1

### Preparation of a catalyst.

Cobalt (II) acetate tetrahydrate dissolved in water was combined with aluminium isopropoxide and homogenized until a gel was obtained. A second solution consisting of orthophosphoric acid and water, and subsequently di-n-propylamine were added under agitation. The composition of the final reaction gel expressed in molar (oxide) ratios was as follows: 0.075 CoO : 1 Al₂O₃ : 1 P₂O₅ : 1 R : 45 H₂O, wherein R is di-n-propylamine.
The reaction gel was then transferred to an autoclave to crystallize at 160 °C for 48 hours. The solid was recovered by filtration, washed with water and dried in air at 120 °C. In order to remove organic moieties occluded in the pores of the solid material obtained, the solid material was calcined in air at 550 °C for 3 hours. The product so obtained had an X-ray diffraction pattern characteristic of highly-crystalline CoAPO-11, and its anhydrous composition expressed in molar ratios was as follows:
(CoO)_{0.02}(Al₂O₃)_{0.24}(P₂O₅)_{0.25}.
The CoAPO-11 powder thus obtained was combined with methanol (10 ml/g), and the slurry was stirred until the colour of CoAPO-11 changed from green to blue indicating reduction to the acidic oxidation state has taken place. The solid was then separated from the liquid phase by filtration, washed with hot water and dried. Before use in a process according to the present invention the CoAPO-11 was intensively mixed with powderized alumina supporting 1% by mass of highly dispersed platinum in a mass ratio of 1:1 on dry basis. The powder mixture so obtained was then pressed, crushed and sieved in order to obtain a 40-80 mesh fraction of pellets of a catalyst A. Finally, catalyst A obtained was calcined by stepwise heating in air at a final temperature of 525 °C for 90 minutes.

### Example 2

### Preparation of a catalyst.

Pellets of a catalyst B were prepared in a similar manner as Catalyst A except that the cobalt (II) acetate tetrahydrate solution was combined with silica sol (Ludox AS) and aluminium propoxide. The composition of the final gel expressed in molar (oxide) ratios was as follows:
0.08 CoO : 0.08 SiO₂ : 0.85 Al₂O₃ : 1 P₂O₅ : 1 R : 45 H₂O, wherein R is di-n-propylamine, whereas the product obtained from the calcination step which was carried out at 550 °C for 3 hours had an X-ray diffraction pattern characteristic of highly-crystalline CoAPSO-11, and its anhydrous composition expressed in molar oxide ratios was as follows:
(CoO)_{0.03}(SiO₂)_{0.03}(Al₂O₃)_{0.23}(P₂O₅)_{0.24}.

### Example 3

### Preparation of a catalyst.

A template-free SAPO-11 material was intensively mixed with powderized alumina supporting 1% by mass of highly dispersed platinum in a mass ratio of 1:1 on dry basis. The powder mixture was then pressed, crushed and sieved in order to obtain a 40-80 mesh fraction of pellets of a catalyst C. Finally, catalyst C obtained was calcined by stepwise heating in air at a final temperature of 525 °C which was maintained for 90 minutes.

### Example 4

### Experiments with catalysts A-C.

Each catalyst was exposed to a hydrogen flow at a pressure of 30 bar and heated at 400 °C for 2 hours. Thereafter, the catalyst was allowed to cool down to the desired reaction temperature. Subsequently, the catalyst was contacted with a feedstock consisting of n-heptane at a temperature of 340 °C, a total pressure of 30 bar, a hydrogen/n-heptane mass ratio of 0.08 and a weight hour space velocity of 2 kg/kg.h.

The results of these experiments are shown in Table 1, from which it will be clear that processes in accordance with the present invention (use of catalysts A and B) are far more attractive in terms of mono-branched C₇ than a process just falling outside the scope of the present invention wherein use is made of catalyst C.

**Table 1**

| Catalyst i-C₇ | Conversion n-heptane (% by mass) | Selectivity (% by mass) |
|---|---|---|
| A | 67 | 94 |
| B | 69 | 96 |
| C | 47 | 85 |

## Claims

1. Process for the isomerisation of a hydrocarbonaceous feedstock substantially boiling in the gasoline range which feedstock comprises linear paraffins having at least five carbon atoms by contacting the feedstock in the presence of hydrogen at elevated temperature and pressure with a catalyst comprising a MeAPO and/or MeAPSO medium-pore molecular sieve and at least one Group VIII metal component, wherein Me is at least Mg, Mn, Co or Zn.

2. Process according to claim 1, wherein Me is at least Co.

3. Process according to claim 1 or 2, wherein the medium-pore molecular sieve(s) has (have) an anhydrous composition which can be expressed in terms of molar oxide ratios as follows: (MeO)ₐ(Al₂O₃)_{b}(P₂O₅)_{c}(SiO₂)_{d}, wherein a ranges from 0.003 to 0.2, b ranges from 0.05 to 0.3, c ranges from 0.05 to 0.3, and d is at most 0.4.

4. Process according to claim 3, wherein a ranges from 0.005 to 0.15, b ranges from 0.05 to 0.27, c ranges from 0.05 to 0.27, and d is at most 0.3.

5. Process according to any one of claims 1-4, wherein the hydrocarbonaceous feedstock substantially comprises linear paraffins having five to ten carbon atoms.

6. Process according to claim 5, wherein the hydrocarbonaceous feedstock substantially comprises linear paraffins having six to eight carbon atoms.

7. Process according to any one of claims 1-6 wherein the catalyst comprises 0.01 to 5% by weight of Group VIII metal based on total catalyst.

8. Process according to any one of claims 1-7 wherein the Group VIII metal component comprises a noble-metal component.

9. Process according to claim 8, wherein the noble-metal component comprises a platinum and/or palladium component.

10. Process according to any one of claims 1-9, wherein the process is carried out at a temperature of 200 to 450 °C, a pressure of up to 100 bar, a space velocity of 0.5 to 10 kg/kg.h and a H₂/feedstock weight ratio of less than 5.

11. Process according to any one of claims 1-10, substantially as described hereinbefore with reference to the non-comparative part of the Examples.
